(19)

Europäisches
Patentamt
European
Patent Office
Office européen
des brevets

(11) **EP 2 052 683 A1**

(12) **EUROPEAN PATENT APPLICATION**

(43) Date of publication:
**29.04.2009 Bulletin 2009/18**

(51) Int Cl.:
***A61B 5/145*** *(2006.01)*    ***G01N 11/00*** *(2006.01)*

(21) Application number: **07020690.9**

(22) Date of filing: **23.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Sensile Pat AG**
**6340 Baar (CH)**

(72) Inventors:
• **Straessler, Sigfrid**
  **1113 St-Saphorin-sur-Morges (CH)**

• **Geiges, Thomas**
  **8707 Uetikon am See (CH)**
• **Kuenzi, Simon**
  **1026 Echandens (CH)**

(74) Representative: **Reuteler, Raymond Werner**
**Reuteler & Cie SA**
**Chemin de la Vuarpillière 29**
**1260 Nyon (CH)**

(54) **Liquid flow sensing system**

(57)    A liquid flow sensing system comprises a pump system configured to deliver a pre-determined volume V of liquid, and a measuring system comprising a chamber connected to the pump system and to an outlet system, the chamber bounded by an elastic membrane configured to be displaced elastically by an amplitude that enables said pre-determined volume V of liquid to be injected in the chamber. The measuring system further comprises a membrane displacement sensor configured to measure at least a portion of a decay characteristic of said membrane displacement amplitude after injection of the pre-determined volume V, into a value that may be used for determining absolute or relative viscosity of the liquid or changes in flow resistance downstream of the sensing system or for determining the presence of gas bubbles in a liquid flow circuit.

FIG. 1

**Description**

The present invention relates to a system for sensing the flow properties or conditions of a liquid, such as flow resistance or presence of gas (e.g. air bubbles) in a liquid supply or circulation system.

Measuring the resistance of a liquid flowing in a liquid supply circuit has uses in many applications such as determining the viscosity of the liquid or determining whether there is leakage in a liquid flow circuit or whether the liquid circuit is blocked or closed in many medical and non medical applications.

In the medical field, an example of an application of liquid viscosity measurement is in determining blood sugar levels based on the viscosity change of solutions sensitive to glucose levels. A liquid flow sensing system is also particularly useful in other medical applications such as to determine whether there is occlusion or leakage in a liquid drug administration device, for example a liquid drug supply and pump system connected to a transcutaneous injection needle.

Sensing systems, particularly in the medical applications field, need to be very reliable, in view of the safety requirements, and often very compact in view of the desire for miniaturization of medical devices, particularly portable medical devices that are carried by patients.

In many applications, there is also a need for disposable devices that are cost effective and that may be disposed of with a low negative environmental impact.

It is an object of this invention to provide a liquid flow sensing system based on sensing liquid flow resistance, that is reliable, efficient, and accurate.

It would be advantageous to provide a liquid flow sensing system that is cost effective, compact and versatile.

It would be advantageous to provide a liquid flow sensing system that may be disposed of with a low negative environmental impact.

It is another object of this invention to provide a reliable, efficient and accurate liquid viscosity measurement system, in particular for medical applications such as blood sugar level monitoring.

It is another object of this invention to provide a liquid flow resistance measuring system for occlusion or leakage detection or for determining the open or closed status of a liquid supply circuit.

It would be advantageous to provide the aforementioned liquid flow resistance measuring systems for medical applications in particular for trans-cutaneous drug administration systems.

It would be advantageous to provide a liquid flow sensing system that is compact, light weight and consumes little power, in particular for incorporation in portable autonomous devices.

Objects of this invention have been achieved by providing the liquid flow sensing system according to claim 1.

Disclosed herein is a liquid flow sensing system comprising a pump system configured to pump a pre-determined volume V of liquid within a range of time $t$, and a measuring system comprising a chamber connected to the pump system and to an outlet system, the chamber bounded by an elastic membrane configured to be displaced a measurable amplitude when the pre-determined volume V of liquid is injected in the chamber. The measuring system further comprises a membrane displacement sensor configured to measure displacement of the membrane, in particular the decay in amplitude of the membrane as it returns from a stressed state to a rest state after injection of the pre-determined volume V. The measuring system further comprises a signal processing circuit configured to process said amplitude decay of the membrane into a value that may be used for determining absolute or relative viscosity of the liquid, or changes in flow resistance downstream of the sensing system, or for determining the presence of gas bubbles in a liquid flow circuit.

The return or relaxation characteristic of the membrane depends on the resistance to flow of the liquid downstream of the chamber which in turn depends on the viscosity of the liquid and of resistance due to fluid circuit geometry or to systems influencing pressure in the liquid flow circuit or its outlet.

In an application for viscosity measurement, the liquid flow sensing system may be provided with a resistive passage, for example in form of a section of capillary extending directly from the outlet of the chamber, or along a section of liquid flow circuit downstream of the chamber.

Also disclosed herein is a method of measuring viscosity of a liquid, comprising providing a chamber bounded by an elastic membrane and connected to an outlet system applying a resistance to liquid flow, injecting a pre-determined volume V of liquid into the chamber, measuring displacement of the membrane, and determining, from at least a section of decay characteristic of the membrane displacement, a value correlated to viscosity of the liquid.

The volume V of liquid is preferably injected (pumped) into the chamber at a rate greater than twice an average rate of exit of the liquid out of the chamber through the flow resistance, more preferably greater than five times the average rate of exit of the liquid out of the chamber through the flow resistance.

As an alternative method, the same system may also be used in the reverse direction, whereby the pump sucks a predetermined volume V out of the chamber, thus creating a pressure drop in the chamber. Accordingly, the elastic membrane in this case displaces inwardly towards the inside of the chamber, whereas the decay behaviour of the elastic membrane returning to its relaxed state may be measured to determine flow resistance of liquid returning to fill the chamber. This alternative is regarded as an equivalent solution to the same technical problem, and for simplicity, only the first mentioned direction shall be described in further detail, without excluding the reverse direction from the ambit of this invention.

Also disclosed herein is a blood sugar level monitoring system comprising a liquid flow sensing system as set forth above, where the outlet system comprises a trans-cutaneous implantable member.

In an embodiment, the trans-cutaneous implantable member comprises a semi permeable membrane allowing passage of glucose molecules therethrough, the sensitive liquid having the property of reversibly changing viscosity as a function of glucose concentration.

In another embodiment, the trans-cutaneous implantable member comprises a porous membrane having a porosity that changes as a function of the concentration of glucose in the medium surrounding the implantable member and configured to allow the liquid of the sensing system to pass therethrough under pressure.

Also disclosed herein is a liquid drug delivery system comprising a transcutaneous injection member connected to a liquid drug supply, and a sensing system as set forth above but adapted for occlusion or leakage detection.

Also disclosed herein is a method of measuring viscosity of a liquid by means of a sensing system comprising a chamber bounded by an elastic membrane and connected to an outlet with a flow resistance portion, comprising the steps of injecting a pre-determined volume V of liquid into the chamber, measuring an amplitude of displacement of the membrane, and determining, from at least a section of decay characteristic of the membrane displacement amplitude, a value correlated to viscosity of the liquid.

Also disclosed herein is a method of detecting leakage or occlusion of a liquid delivery circuit, by means of a sensing system comprising a chamber bounded by an elastic membrane and connected to an outlet with a flow resistance portion, comprising the steps of injecting a pre-determined volume V of liquid into the chamber, measuring an amplitude of displacement of the membrane, and determining, from at least a section of decay characteristic of the membrane displacement amplitude, a value correlated resistance to flow of the liquid.

Further objects, advantageous features and aspects of the invention will be apparent from the claims and following detailed description with reference to the figures in which:

Figure 1 shows a schematic cross-sectional representation of an embodiment of a liquid flow sensing system according to this invention;

Figure 2 shows in exploded perspective a schematic illustration of an embodiment of part of a sensing system according to this invention;

Figure 3 illustrates a graph of a pump and relaxation cycle of a sensing system according to this invention;

Figure 4a illustrates a cross-sectional view of implementation of the invention in a blood sugar level monitoring system according to a first embodiment;

Figure 4b illustrates a cross-sectional view of an implantable member of the system of figure 4a;

Figure 5a illustrates a cross-sectional view of implementation of the invention in a blood sugar level monitoring system according to a second embodiment ;

Figure 5b is a perspective view of part of the system of figure 5a;

Figure 6 is a graphical representation of response signals of the liquid flow sensing system according to another embodiment for occlusion or leakage detection;

Figure 7a illustrates a graph showing possible diameters of the membrane as a function of the pumped volume under certain conditions;

Figure 7b illustrates a graph showing maximal amplitude of the membrane as a function of the pumped volume under certain conditions;

Figure 7c illustrates a graph showing possible choices for the thickness of the membrane $h$ as a function of the membrane diameter for a certain pumped volume;

Figure 7d illustrates a graph showing membrane amplitude as a function of the number of pumped cycles, in view of determining creep resistance of the membrane material;

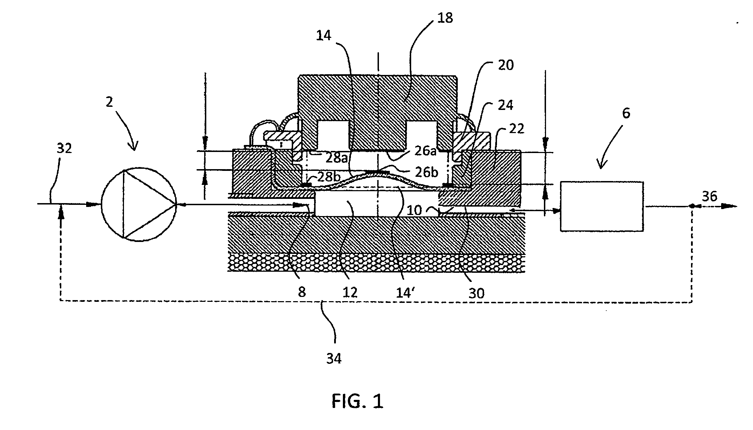

Referring to the figures, in particular figures 1-3, a liquid flow sensing system 1 comprises a pump system 2, a liquid flow measuring system 4 connected to the pump system, and an outlet system 6 connected to the measuring system. The outlet system may have different configurations depending on the application, for example the outlet system could comprise a liquid uptake reservoir, or could feed into a re-circulation circuit 34 for re-circulating liquid flowing through the system back to the pump system, or comprise a catheter or needle for drug delivery, or an implantable member with porous membrane that changes its porosity, or a flow resistance, or simply an outlet to discharge the liquid.

The measuring system 4 comprises a measuring system inlet 8 connected to the pump system 2, an outlet 10 connected to the outlet system 6, a chamber 12 bounded at an open-end by an elastic membrane 14, a membrane displacement sensor 16, and a measurement signal processor 18.

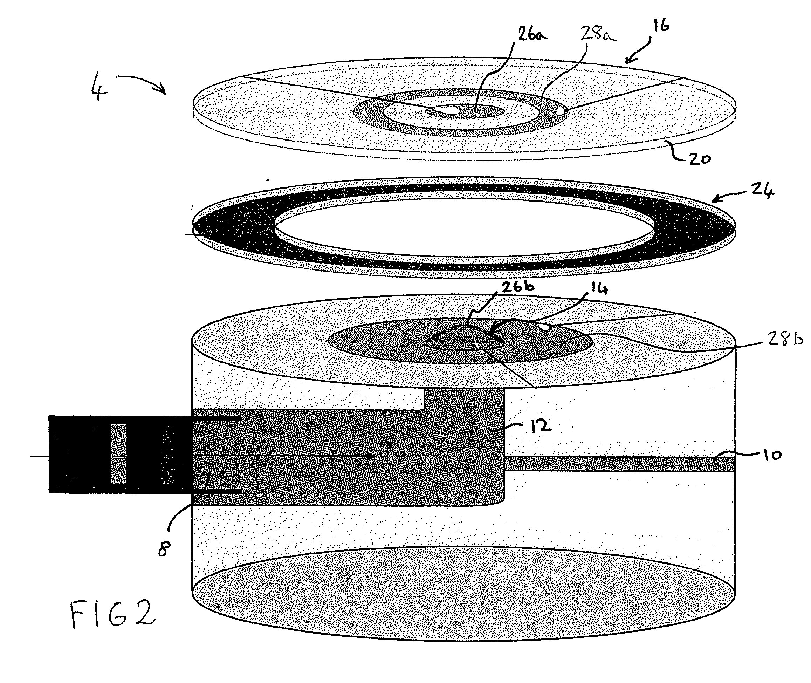

In the embodiment shown in figures 1 and 2, the membrane displacement sensor is based on the principle of capacitive measurement, and comprises a first capacitor electrode 26a, for example in the form of a conductive layer that may be in the general form of a disk, facing a second capacitor electrode 26b in the form of a conductive layer in or on the elastic membrane 14. The capacitor electrode layers 26a, 26b are connected to the measurement signal processor 18. The opposing capacitor electrodes 26a, 26b are separated by a spacer 24, in this embodiment positioned between a circuit board 20 and a peripheral edge of the elastic membrane 14. A reference capacitor 28a, 28b formed by electrode layer rings on the membrane and signal processor co-axially surrounding the respective capacitive layers 26a, 26b. The reference capacitor layers are positioned on surface portions that are subject to no displacement such that they provide a reference value to compensate in particular for external electrical field disturbances, or temperature related variations, but also changes in material properties due to wear in prolonged use.. The spacer may be provided with electrical conductors (not shown) for interconnecting the electrodes on the elastic membrane to the circuit board,

Displacement of the elastic membrane is measured by determining the electrical capacitance value between the electrodes 26a, 26b.

The measuring system may be based on other distance measuring principles, an alternative e.g. being by means of a laser diode emitter and receiver system or other optical systems that could also be mounted over the membrane spaced at a certain distance therefrom whereby the system measures the light travel sent from the emitter, reflected off the centre top of the membrane back to the receiver. Alternatively, hall effect sensors, or eddy current sensors, or acoustic measuring principle (i.e.ultrasound), or pressure sensors could be employed.

The pump system 2 comprises a pump that is configured to pump a defined volume V rapidly in a discrete dose. A pump capable of delivering small volumes of liquid accurately and rapidly is known from WO 2007 074363 and may advantageously be incorporated in the liquid flow sensing system according to this invention. The pump system could also comprise a reservoir under pressure and a valve on the input line 8 configured to open and close to allow injections of the predetermined and controlled volume V of liquid into the chamber 12.

The basic functioning of the sensing system according to the invention is as follows:

■ A predetermined volume V of a liquid is pumped into the chamber within a time scale $T_{inp}$ which is short compared to the output time scale $T_{out}$

$$T_{inp} << T_{out}$$

■ The deformation of the membrane generates a pressure difference $p$ which initiates a flow of the liquid through a flow resistor, such as a resistance cannula or capillary downstream of the chamber, or a resistance due to an external influence;

■ The decay of the amplitude a of displacement of the membrane is measured which is related to the rate of flow of liquid out of the chamber, which in turn is indicative of the flow resistance.

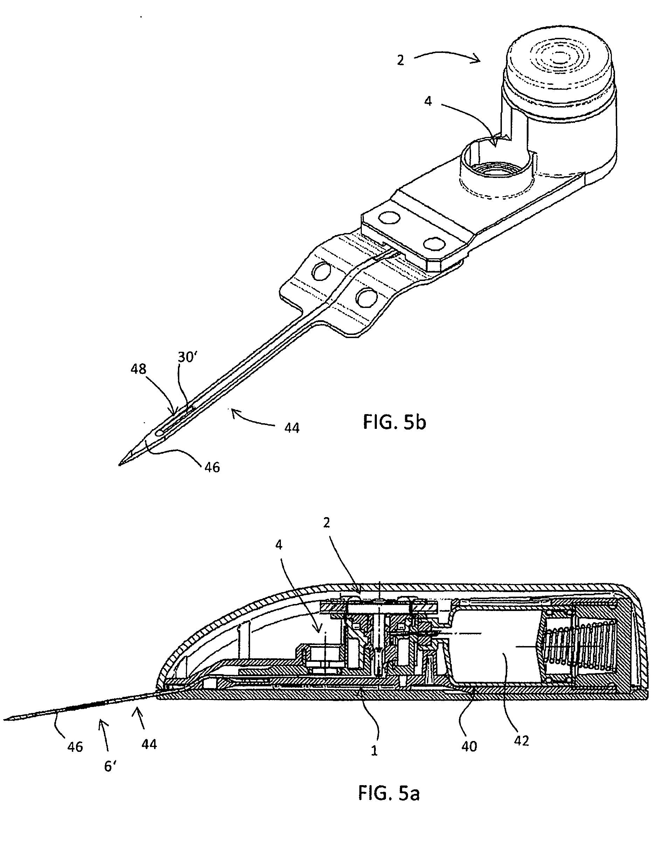

Depending on the particular application, the membrane must store a certain volume V, for instance for the application illustrated in figures 5a, 5b around 100nl, and should generate a certain pressure, for instance for the application illustrated in figures 5a, 5b, around 80mbar. In addition the particular application requires a displacement amplitude relaxation or decay time $T_{out} = \tau$ which must reside in some time window, for instance for the application illustrated in figures 5a, 5b, around 1 second to 3 seconds.

The above-described functioning principle of the invention may be adapted for use in various applications, including measuring liquid viscosity, or measuring flow resistance to determine leakage or occlusion in a liquid flow circuit, or detecting the presence of air or other gas bubbles in a liquid flow circuit.

It is advantageous for many applications to produce parts of the sensing system in plastics material in view of reducing material and manufacturing costs as well as producing a light weight system that may also be easily disposed of. Plastics are also preferred in view of their advantageous Youngs modulus range for application in this invention. In many medical field applications, and in other applications, the volumes of liquid to be pumped are very small, in the range of 10 nano litres to 10 micro litres such that the diameter of the membrane would typically lie in a range of 2 to 10 mm.

For reliable and sufficiently accurate measurement of the membrane displacement, the elastic properties of the membrane should satisfy certain criteria, in particular the young's modulus should lie within a certain range depending on the desired pressure, and the membrane should have a low creep characteristic for the maximum value of membrane displacement expected. An example of the relationships between the magnitudes of the various parameters defining the membrane as a function of a pumped discrete volume V lying in a range of 10 nanolitres to 2 microlitres is set forth hereafter.

According to Timoshenko et al. (Timoshenko, S.P., Woinowsky-Krieger, S., 1959. Theory of Plates and Shells. McGraw-Hill, New York.) it follows that in the linear approximation the following relationship between the magnitudes of relevant parameters can be established:

$$V = \frac{1}{3} a\pi \left(\frac{\phi}{2}\right)^2 \qquad\qquad \text{(i)}$$

$$a = \frac{3P\phi^4(1-\nu^2)}{256 \cdot Y \cdot h^3} \qquad\qquad \text{(ii)}$$

$$\varepsilon = \frac{3P\phi^2(1-\nu^2)}{16 \cdot h^2 \cdot Y} \qquad\qquad \text{(iii)}$$

where:

| | |
|---|---|
| V | injected volume |
| p | pressure in the chamber |
| a | amplitude of displacement at centre of membrane |
| Y | Young's module of membrane material |
| ν | Poisson ratio |
| h | membrane thickness |
| ϕ | diameter of membrane |
| ε | maximal strain (creep is negligible below this strain) |
| ζ = a/h | limit up to which a(p) is approximately a linear function |

In order to ensure repeatable and accurate measurement it is important that creep of the membrane under stress is low. This can be achieved by choosing a low value for the maximal strain ε. Dependent on the material, a reasonable value for the strain is: ε < 0.003, and
for a linear relation between *a* and *p*: ξ< 0.45.

*Injected Volume*

The first step is to choose the pumped (injected) volume. Let us assume applications with injected volumes between 10 nl and 2 μl.

*Membrane Diameter*

After some calculation it follows from (i),(ii) and (iii) that the lower limit of the diameter is determined by $\varepsilon$ and $\xi$:

$$\phi(V) > \frac{48^{1/3}}{\pi^{1/3}(\varepsilon\xi)^{1/6}} V^{1/3}.$$ (iv)

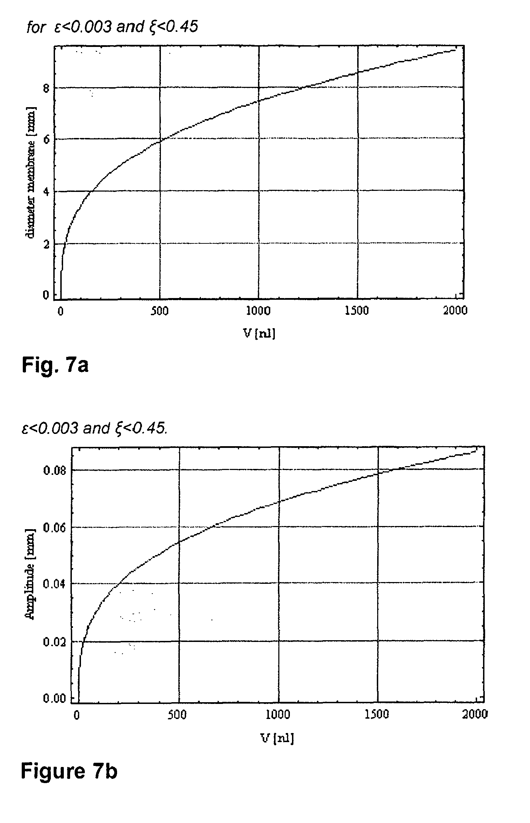

The plot for the conditions $\varepsilon < 0.003$ and $\xi < 0.45$ is shown in Fig. 7a.
The chosen diameter depends on the application. A larger value than the one given by relation (iv) is however normally not convenient, because at a fixed volume the amplitude decreases with increasing diameter of the membrane (see equation (i)).

*Membrane Displacement Amplitude*

When the diameter is chosen, the amplitude is given by equation (i).
The maximal amplitude as a function of the volume for $\varepsilon < 0.003$ and $\xi < 0.45$ is plotted in figure 7b.

*Thickness of the membrane*

At this point the volume and the diameter of the membrane have been chosen, which gives us a maximal amplitude *a*.
The thickness of the membrane *h* is limited by two conditions. The lower limit

$$\frac{12V}{\pi\phi^2\xi} < h$$ (v)

is given by $\xi = a/h$ (follows from equation (i)).
If we choose lower values for h, the relation of a(p) starts to be non linear.
The upper limit

$$h < \frac{\pi\varepsilon\phi^4}{12\cdot16V}$$ (vi)

is determined by the maximal strain $\varepsilon$ (follows from equations (i), (ii) and (iv)).

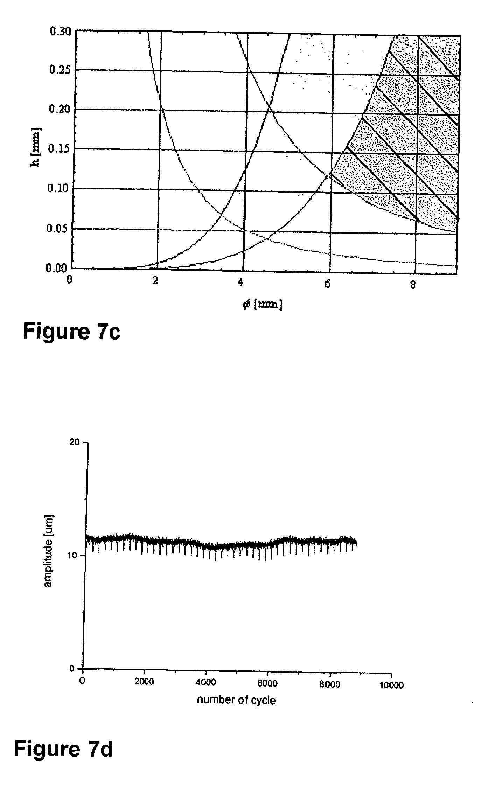

Figure 7c shows the possible intervals in which *h* can be chosen. The possible choices for the thickness of the membrane *h* are shown as function of the membrane diameter for a volume of 100 nl (plain shaded area) and a volume of 500 nl (shaded and line hatched area).

*Young's modulus and the Poisson ratio*

At this point one is still free to choose the Young's modulus of the membrane. The choice depends on the desired pressure in the chamber. The relation between the Young's modulus and the pressure *p* is given by

$$Y = \frac{3(1-v^2)\xi}{\varepsilon^2} p.$$ (vii)

This means that the ratio *Y/p* depends only on $\xi$, $\varepsilon$ and *v* and is independent of the pumped volume.
Thus for $\varepsilon = 0.003$, $\xi = 0.45$ and a typical value of *v* = 0.4, we get the simple equation

$$Y = 1.26 \cdot 10^5 \, p \, .$$ (viii)

(that is $Y$ = 630 N/mM$^2$ for $p$ = 50 mbar or $Y$ = 12 600 N/mm$^2$ for $p$ = 1 bar.) The values for $\varepsilon$ and $\xi$ can however vary from one application to the other.

*Creep*

The creep has to be low enough, such that the Young's modulus of the membrane does not change during the application (in the limits of the desired accuracy). Fig. 7d shows the amplitude variation of a thermoplastic PBT (polybutylene terephthalate) membrane (from GE Plastics named Valox FR1-1001) after several thousands of pump cycles. One cycle is formed by a pump cycle which delivers a predefined volume of liquid with a maximum of 200 mbar. After delivery of liquid by the pump cycle, the pressure decreases exponentially with a relaxation time of 1.5 s ($\phi$ = 3mm, $h$ = 0.075 mm, $v \approx 0.4$, room temperature). As the amplitude stays constant in the desired accuracy, we conclude that the Young's modulus does not significantly change and that the creep is negligible. For the PBT membrane it was found that the maximal strain for an application of this type is approximately 0.3 %, i.e. $\varepsilon$ < 0.003.

**Example**

The following example illustrates how parameters may be chosen for a certain application:

I. For a certain application assume that one wants to pump the volume V = 100 nl.
II. To minimize creep assume that strain should be no greater than 0.3 % => $\varepsilon$ = 0.003.
III. In order to be in the linear range, we chose $\xi$ < 0.45.
IV. With equation (iv) we calculate for the diameter $\phi$>3.46mm. We choose $\phi$ = 3.5 mm.
V. From equation (i) we know that the amplitude should not exceed 31 $\mu$m.

VI. The thickness $h$ lies in the interval $69.3 \, \mu m = \dfrac{12V}{\pi \phi^2 \xi} < h < \dfrac{\pi \varepsilon \phi^4}{12 \cdot 16V} = 73.7 \, \mu m$.

We choose h = 70 $\mu$m.
VII. If the membrane has a Poisson ratio of v = 0.45 and if the pressure is desired to be at 100 mbar, then the Young's modulus of the membrane should be Y=1.3 GPa.

For a particular application the parameters should be chosen such as to give an optimal performance of the device. An overview of examples of practical values is given in Table 1 below.

*Table 1 - example of range of values of various parameters of a fluid flow sensing device according to the invention*

| symbol | name | typical range of values | example of the order of magnitude of values for a device according to fig. 5a-5b |
|---|---|---|---|
| $\phi$ | diameter membrane | 1 - 10 mm | 3mm |
| $h$ | thickness of membrane | 10 - 200 $\mu$m | 60$\mu$m |
| $Y$ | Young's modulus | 0.001 - 100 GPa | 1.0GPa |
| $v$ | Poisson ratio | 0.3 - 0.48 | 0.46 |
| $L$ | length of cannula | 1 - 10 mm | 5mm |
| $d$ | diameter of cannula | 10 - 2000 $\mu$m | 60$\mu$m |
| $V$ | volume | 10 - 1000 nl | 100nl |
| $p$ | pressure | 90 - 500 mbar | 80mbar |
| $\tau$ | relaxation time | 0.01 - 10 s | 1-3 s |
| $\eta$ | viscosity | 0.009 - 0.1 Pas | 0.014 Pas |

As discussed above, for the measurement system to function optimally for an application as shown in figures 5a and 5b, the membrane needs to have an elastic displacement range that allows it to expand reversibly without measurable creep with an injection of a volume of liquid, preferably in a range of 10nl to 2µl, and that generates a pressure, preferably in a range of 10 mbar to 500 mbar that enables the membrane relaxation displacement to be measured. The membrane may advantageously be made of a polymer material or a multilayer sheet of polymer materials, such as polyesters, polyimides or polycarbonates, certain of which advantageously have the desired properties of creep and Young's Modulus. Certain polyesters, such as PBT, show good creep properties, as do polycarbonates such as Lexan from GE plastics, or polyimides such as Kapton from DuPont, and may be easily glued or welded to many other thermoplastic polymers. The housing of the chamber may advantageously also be made of a polymer, for example by injection moulding, with the inlet and resistive outlet orifice integrally formed therewith. The membrane may be in the form of a disc cut from a sheet of laminated material or multilayer materials and clamped, bonded or welded at its periphery to the chamber housing. The membrane may also be integrally formed with the chamber housing material, by injection molding, or milling an appropriate hole out of a cylindrical starting material.

Forming of the measuring system housing and membrane from plastics materials enables the measuring system to be compact, lightweight and economical to manufacture in series production. Moreover the materials may be chosen also for their ease of recycling and disposal with minimal environmental impact. Plastics material also provides a high design flexibility that allows incorporation of the measuring system with a pump system or drug delivery device, integrally therewith, or as a separate component assembled thereto.

The membrane could also be made of other materials that exhibit the creep, Young's modulus and other properties discussed above suitable for the range of operation of the measuring system.

Referring now to figures 5a and 5b, an example of an application in the medical field of the liquid flow sensing system according to this invention is illustrated.

Figures 5a, 5b illustrate a blood sugar monitoring system comprising a sensitive solution supply system 40 with a reservoir of sensitive solution 42 connected a liquid flow sensing system 1 according to this invention with a micropump 2 and a measuring system 4 connected at its outlet to an outlet system 6' in the form of a dialysis member 44 having a needle section 46 adapted to be inserted trans-cutaneously. The dialysis member comprises a section with a semi-permeable membrane 48 intended to be in contact with a patient's interstitial liquid (in order to determine a patient's blood glucose level, usually the glucose level in the interstitial liquid is measured, which is influenced by the blood glucose level, whereas with a certain lag time the glucose level in the interstitial fluid equilibrates to the blood glucose level) whereby the semi-permeable membrane allows glucose diffusion therethrough. This sensitive solution is known in the art as is the membrane and dialysis needle and thus shall not be described in detail in the present.

Depending on the patient's blood sugar level, more or less glucose diffuses through the semi-permeable membrane into the sensitive solution, the viscosity of which varies as a function of the glucose level. Thus, there is a correlation between the viscosity of the sensitive solution and the patient's blood sugar level. Measurement of the sensitive solution viscosity can thus be used to determine the blood sugar level.

In the embodiment shown, the liquid flow sensing system 1 is upstream of the semi-permeable membrane 48 whereas the flow resistance section in the form of a capillary channel section 30' is positioned downstream of the semi-permeable membrane.

The outlet system comprises a waste reservoir for discharging used sensitive solution. Alternatively, it is however possible to provide a closed circuit without a waste chamber by re-circulating the sensitive solution. In effect, the semi-permeable membrane allows glucose diffusion in both directions, allowing an equilibrium between the glucose level in the patient's blood and in the sensitive solution to be achieved. The sensitive solution with glucose content may thus be re-circulated and re-used.

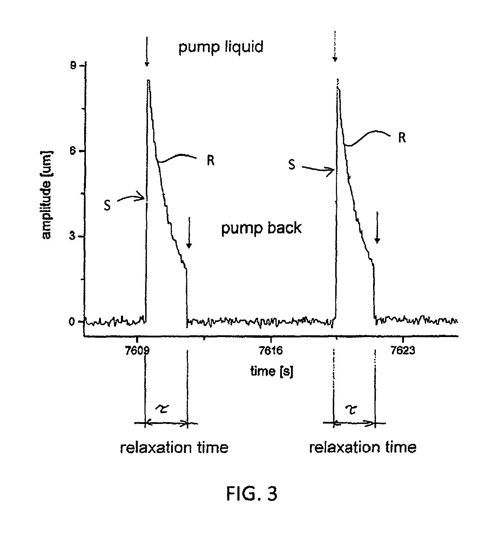

Blood sugar level is thus measured with the apparatus of figures 5a, 5b by pumping a certain volume of the sensitive fluid in the section of semi-permeable membrane and allowing it to stand still for sufficient time to allow diffusion of glucose through the membrane to substantially achieve an equilibrium level between glucose concentration on either side of the membrane. Subsequently, a pre-determined volume of sensitive solution is pumped with the pump into the chamber 12 of the measuring system. The pre-determined volume of liquid is injected rapidly by the pump in comparison to the flow rate of the sensitive fluid through the resistive capillary channel section 30'. The amplitude of membrane displacement is measured, as illustrated by the signal *S* in figure 3, the relaxation characteristic *R* being representative of the liquid viscosity. The amplitude decay characteristic may thus be correlated with a stored or computed value enabling correlation of the amplitude decay characteristic with a blood sugar level.

As the compressibility of the air bubble containing liquid changes in the presence of air, the amount of air in the system can be calculated from the maximal displacement of the membrane. Even though the system is disturbed by air, a correct value may be calculated by incorporating the influence of an air bubble on the measurement.

If a plurality of successive viscosity measurements are desired, it is possible to reverse the pump operation after a pre-determined relaxation time to remove the remaining pumped volume, thus bringing the membrane to its unsolicited state, ready for a new measurement cycle, as illustrated in figure 3.

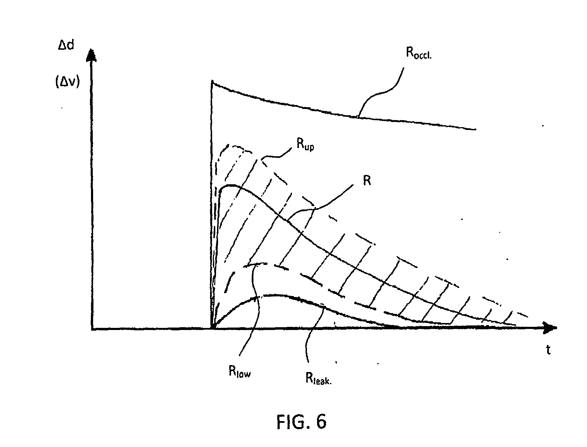

The embodiment of figures 5a, 5b may be adapted for liquid drug administration, by replacing the dialysis needle with an injection needle. In such case, the liquid flow sensing system according to the invention may function as a leakage or occlusion detection system. In the afore-mentioned application, the system works in a similar way to that described above whereby a pre-determined volume of liquid is pumped by the micropump and subsequently the membrane displacement is measured. The liquid flow sensing system may be provided with an internal liquid flow resistance which adds to the resistance of the trans-cutaneous injection system depending on the need to adjust the response of the sensing system. It may also be possible to avoid any resistance in the sensing system and rely solely on the resistance to the liquid flow due to the trans-cutaneous injection outlet. If the injection outlet is blocked (occlusion) for example due to a blood clot or for other reasons, the response $R_{occl}$ will have a long relaxation time as shown in figure 6, and if there is no resistance, such as in the case of the needle being removed (leakage), the relaxation time will be short and the peak displacement of the response $R_{leak}$ may be very low. Correct operation may be defined by setting upper and lower limits $R_{up}$, $R_{low}$, the expected response $R$ lying between these limits whereby a response curve above or below the limits triggers an alarm.

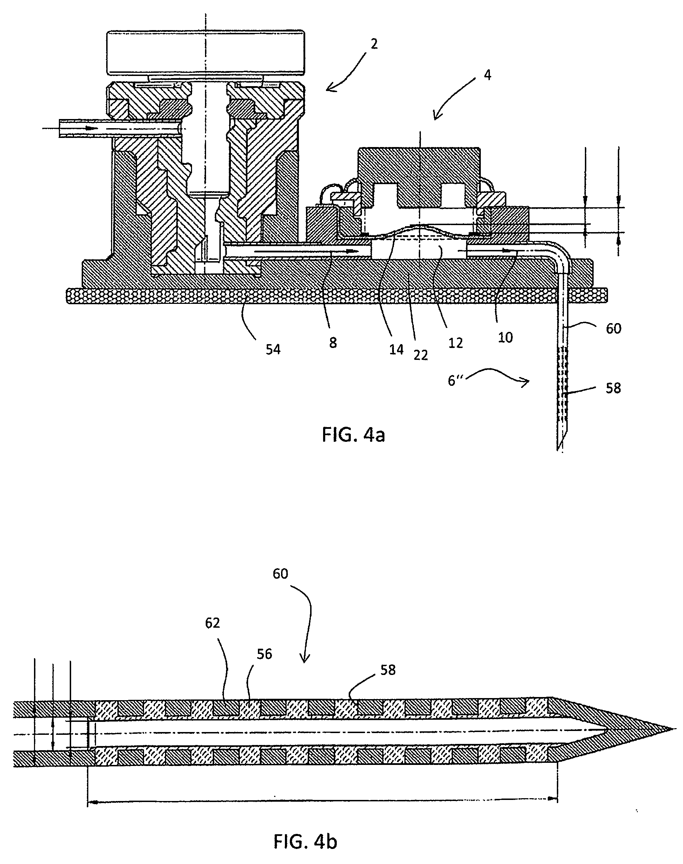

Figure 4a and 4b illustrate a blood sugar monitoring system according to another embodiment, comprising a liquid flow sensing system 1 according to this invention with a pump system 2 comprising a reservoir of liquid and a micropump, and a measuring system 4 connected to an outlet system 6" in the form of an implantable member 60 adapted to be inserted trans-cutaneously. The pump system and measuring system may be housed in a housing that may be carried or worn by a patient, and for instance comprises an adhesive base 54 for application on the patient's skin.

The implantable member 60 may be in the form of a needle that extends from the base of the housing, or separated from the housing and interconnected therewith via a flexible catheter. The implantable member comprises a section with a porous membrane 56 intended to be in contact with a patient's blood whereby the porous membrane allows the liquid of the sensing system to pass therethrough under pressure.

As the concentration of glucose in the medium surrounding the implantable member changes, the porosity of the porous membrane contained in the porous membrane 56 changes. Preferably the semi-permeable membrane contains immobilized concanavalin A and dextran molecules to form a hydrogel. Said hydrogel is capable of reversibly changing its structure depending on the glucose concentration present. Free glucose molecules competitively and specifically bind to immobilized concanavalin A molecules. A raise in glucose concentration will raise the number of concanavalin A binding sites occupied with glucose molecules and thus enlarging the size of the pores present in the hydrogel. If the glucose concentration decreases, glucose bound to concanavalin A will be replaced by dextran molecules which form an interlinked web-like structure, thus reducing the size of the pores present in the hydrogel. The response time for the porous membrane to reach equilibrium of glucose concentration according to the surrounding solution to 95 percent will typically measure 30 seconds.

Given a change in glucose concentration in the solution surrounding the implantable member between 0 mmol/l and 30 mmol/l (the normal bandwidth of glucose concentration in the human blood is 4 mmol/l to 8 mmol/l), the effective pore size in the porous membrane would typically range from 50 nm to 100 nm in diameter. Depending on the flow resistance subject to the porosity of the porous membrane in the implantable member, the decay behavior of the flexible membrane varies, as discussed previously. The higher the flow resistance through the porous membrane, the longer it takes for the flexible membrane to reach its original position after displacement caused by the liquid delivered by the pressure generating means. While the flexible membrane of the measuring system returns back to its rest position, liquid leaves the sensor chamber through the outlet channel 10 und thus the liquid is conveyed towards the implantable member. The blood glucose concentration can thus be determined with the liquid flow sensing system as previously described by measuring the flow resistance through the porous membrane. This can preferably be done by using a physiological saline solution as liquid.

In order to determine a standard value to which the measurements of the glucose concentration can be compared, a succession of discrete volumes of liquid are injected, for example 50 nano liters separated by 3 seconds, i.e. as soon as the flexible membrane has reached its relaxed state, a new discrete volume of liquid is delivered. Thus, a succession of fluid resistance measurements is performed rapidly, without long pauses in-between the measurements. After a number of units are delivered, for example 5 units, the porosity of the porous membrane reaches a value that is determined by the glucose concentration of the liquid contained in the medical device, which is a known value and thus can be used as a reference value. The first unit is used to measure the glucose concentration in the solution surrounding the implantable member, the second unit will replace for example 80 percent of the equilibrated liquid in the needle lumen (in this example, the pump volume is equal to the active volume in the needle), and after the third unit, only about 3 percent of the equilibrated liquid remains in the responsive region of the needle. Therefore, a small number of units pumped through the porous membrane, e.g. 5 units, rinse the porous membrane and are enough to obtain a reference value for calibration.

In order to calibrate the glucose measurement, simply the first measurement (which measures the glucose concentration of the solution surrounding the implantable member) of the above described series of measurements can be compared to the last measurement (which measures the glucose concentration of the liquid present in the medical device).

After a pause, for example for 30 seconds, the porosity of the porous membrane has reached a value that is again determined by the glucose concentration surrounding the membrane. After about 30 seconds, given a pore size of 50nm to 100nm in the porous membrane, the liquid in the responsive part of the needle will reach almost the same glucose concentration as the concentration that is present in the surrounding solution. Therefore, a new measuring cycle with integrated calibration as described above may be commenced.

Typically, a new measuring cycle is not required more frequently than every ten minutes. The lag times as described above will thus not unduly restrict the application of the present invention for continuous glucose monitoring.

The hydrogel in the implantable member may be held in a tubular member 62 comprising slots or holes 58. The membrane advantageously is supported by the tubular member 62 which may be made of any firm biocompatible material, such as metallic, plastic, or ceramic materials.

A hydrogel suitable for the present invention has been described in detail: Tang et al. report the synthesis of a mechanically and chemically stable, glucose responsive hydrogel membrane, which can be cast in a number of mechanical forms. The response to changes in glucose concentration was demonstrated to be reversible in both directions, i.e. the transitions between gel and sol phase. Furthermore, the hydrogel showed negligible leakage of Concanavalin A over extended periods. The use of two dextran species with different molecular weights allowed greater control over the gel structure, such that property changes can be restricted to changes in internal porosity of the hydrogel (Tang et al. 2003: A reversible hydrogel membrane and delivery of macromolecules. Biotechnology and Bioengineering, Vol. 82, No. 1, April 5, 2003).

Advantageously, concanavalin A is immobilized within the hydrogel, so that concanavalin A is prevented from entering the patient's body as it has been reported to have a toxic effect on humans. Methods to immobilize concanavalin A have been reported: Miyata et al. report the synthesis of a concanavalin A copolymerized glucosyloxyethyl methacrylate (GEMA) hydrogel, from which concanavalin A did not leak out and thus a reversible change in porosity of the porous membrane can be achieved (Miyata et al. 2004: Glucose-responsive hydrogels prepared by copolymerization of a monomer with Con A. Journal Biomaterial Science Polymer Edition, Vol. 15, No. 9, pp 1085 - 1098, 2004). Kim and Park reported the immobilization of concanavalin A to glucose-containing polymers (Kim J.J. and Park K. 2001: Immobilization of Concanavalin A to glucose-containing polymers. Macromolecular Symposium, No. 172, pp 95 - 102, 2001).

## Claims

1. A liquid flow sensing system comprising a pump system configured to deliver a pre-determined volume V of liquid, and a measuring system comprising a chamber connected to the pump system and to an outlet system, the chamber bounded by an elastic membrane configured to be displaced elastically by an amplitude that enables said pre-determined volume V of liquid to be injected in the chamber, the measuring system further comprising a membrane displacement sensor configured to measure at least a portion of a decay characteristic of said membrane displacement amplitude after injection of the pre-determined volume V, into a value that may be used for determining absolute or relative viscosity of the liquid or changes in flow resistance downstream of the sensing system or for determining the presence of gas bubbles in a liquid flow circuit.

2. Sensing system according to claim 1 wherein the elastic membrane is made of a polymer.

3. Sensing system according to claim 2 wherein the polymer is polyester, polyimide, or polycarbonate.

4. Sensing system according to any one of the preceding claims wherein the membrane is a sheet welded or glued to a housing of the chamber.

5. Sensing system according to any one of the preceding claims wherein the chamber is formed in a housing of thermoplastic material.

6. Sensing system according to any one of the preceding claims wherein the material of the elastic membrane has a Young's Modulus within a range of 0.1 Gpa to 10 GPa

7. Sensing system according to any of the preceding claims wherein the maximal strain applied to the elastic membrane is lower than 0.01.

8. Sensing system according to any one of the preceding claims configured for use in measuring viscosity of a liquid wherein the measuring system comprises a capillary section between the chamber and outlet system for applying a resistance to flow of the liquid out of the chamber.

9. Blood sugar level monitoring system comprising a sensing system according to any one of the preceding claims, wherein the outlet system comprises a trans-cutaneous implantable member.

10. Blood sugar level monitoring system according to the preceding claim wherein the trans-cutaneous implantable member comprises a semi permeable membrane allowing passage of glucose molecules therethrough, and wherein said liquid has a property of reversibly changing viscosity as a function of glucose concentration.

11. Blood sugar level monitoring system according to claim 9 wherein the trans-cutaneous implantable member comprises a porous membrane having a porosity that changes as a function of the concentration of glucose in the medium surrounding the implantable member and configured to allow the liquid of the sensing system to pass therethrough under pressure.

12. Liquid drug delivery system comprising a sensing system according to any one of the preceding claims 1-7 configured for occlusion or leakage detection, and an outlet system comprising a trans-cutaneous injection member.

13. Method of measuring viscosity of a liquid by means of a sensing system comprising a chamber bounded by an elastic membrane and connected to an outlet with a flow resistance portion, comprising the steps of injecting a pre-determined volume V of liquid into the chamber, measuring an amplitude of displacement of the membrane, and determining, from at least a section of decay characteristic of the membrane displacement amplitude, a value correlated to viscosity of the liquid.

14. Method of detecting leakage or occlusion of a liquid delivery circuit, by means of a sensing system comprising a chamber bounded by an elastic membrane and connected to an outlet with a flow resistance portion, comprising the steps of injecting a pre-determined volume V of liquid into the chamber, measuring an amplitude of displacement of the membrane, and determining, from at least a section of decay characteristic of the membrane displacement amplitude, a value correlated resistance to flow of the liquid.

15. Method of determining blood sugar level by means of a system according to claim 9, comprising the steps of injecting a pre-determined volume V of liquid sensitive to glucose concentration into the chamber, measuring an amplitude of displacement of the membrane, and determining, from at least a section of decay characteristic of the membrane displacement amplitude, a value correlated to viscosity of the liquid and thereby glucose concentration.

16. Method of determining blood sugar level by means of a sensing system comprising a chamber bounded by an elastic membrane and connected to an outlet system with an implantable porous membrane having a porosity varying as a function of glucose concentration, comprising the steps of injecting a pre-determined volume V of liquid into the chamber, measuring an amplitude of displacement of the membrane, and determining, from at least a section of decay characteristic of the membrane displacement amplitude, a value correlated to flow resistance of the liquid through the porous membrane and thereby glucose concentration.

17. A method according to claim 15 or 16 further including a calibration step comprising rinsing the porous membrane by pumping said liquid through the porous membrane and subsequently measuring a flow resistance of the liquid through the porous membrane.

FIG. 1

FIG 2

FIG. 3

FIG. 4a

FIG. 4b

FIG. 5b

FIG. 5a

FIG. 6

*for ε<0.003 and ξ<0.45*

**Fig. 7a**

*ε<0.003 and ξ<0.45.*

**Figure 7b**

**Figure 7c**

**Figure 7d**

**European Patent Office**

**EUROPEAN SEARCH REPORT**

Application Number

EP 07 02 0690

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 195 01 159 A1 (EHWALD RUDOLF PROF DR SC NAT [DE] EHWALD RUDOLF [DE]) 11 July 1996 (1996-07-11) * column 1, line 53 - column 5, line 31 * ----- | 1-10, 12-15,17 | INV. A61B5/145 G01N11/00 |
| X | US 4 926 682 A (HOLM-KENNEDY JAMES W [US] ET AL) 22 May 1990 (1990-05-22) * column 3, line 8 - column 4, line 62 * ----- | 1-8,13, 17 | |
| | | | TECHNICAL FIELDS SEARCHED (IPC) A61B G01N |

The present search report has been drawn up for all claims

| Place of search | Date of completion of the search | Examiner |
|---|---|---|
| Munich | 25 April 2008 | Martelli, Luca |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                    EP 07 02 0690

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2008

| Patent document cited in search report | | Publication date | Patent family member(s) | | Publication date |
|---|---|---|---|---|---|
| DE 19501159 | A1 | 11-07-1996 | US | 6210326 B1 | 03-04-2001 |
| US 4926682 | A | 22-05-1990 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007074363 A

**Non-patent literature cited in the description**

- **TIMOSHENKO, S.P. ; WOINOWSKY-KRIEGER, S.** Theory of Plates and Shells. McGraw-Hill, 1959
- **TANG et al.** A reversible hydrogel membrane and delivery of macromolecules. *Biotechnology and Bioengineering,* 05 April 2003, vol. 82 (1
- Glucose-responsive hydrogels prepared by copolymerization of a monomer with Con A. **MIYATA et al.** Journal Biomaterial Science Polymer. 2004, vol. 15, 1085-1098
- **KIM J.J. ; PARK K.** Immobilization of Concanavalin A to glucose-containing polymers. *Macromolecular Symposium,* 2001, vol. 172, 95-102